Europäisches Patentamt

(19) European Patent Office

Office européen des brevets

(11) EP 0 587 461 B1

(12) EUROPEAN PATENT SPECIFICATION

(45) Date of publication and mention
of the grant of the patent:
16.12.1998 Bulletin 1998/51

(51) Int. Cl.⁶: **A61F 2/06**, A61L 27/00

(21) Application number: 93401516.5

(22) Date of filing: 14.06.1993

(54) **Artificial blood vessel**

Künstliches Blutgefäss

Vaisseau sanguin artificiel

(84) Designated Contracting States:
BE DE FR GB IT NL SE

(30) Priority: 12.06.1992 JP 153301/92

(43) Date of publication of application:
16.03.1994 Bulletin 1994/11

(73) Proprietor:
TERUMO KABUSHIKI KAISHA
Tokyo (JP)

(72) Inventors:
• Nagai, Hirofumi,
c/o Terumo Kabushiki Kaisha
Ashigarakami-gun, Kanagawa-ken (JP)
• Kobarai, Yuki,
c/o Terumo Kabushiki Kaisha
Ashigarakami-gun, Kanagawa-ken (JP)
• Takahashi, Makoto,
c/o Terumo Kabushiki Kaisha
Ashigarakami-gun, Kanagawa-ken (JP)
• Ezaki, Yuzo,
c/o Terumo Kabushiki Kaisha
Ashigarakami-gun, Kanagawa-ken (JP)

(74) Representative:
Gillard, Marie-Louise et al
Cabinet Beau de Loménie
158, rue de l'Université
75340 Paris Cédex 07 (FR)

(56) References cited:
EP-A- 0 122 744          EP-A- 0 146 794
EP-A- 0 448 840          EP-A- 0 552 474
DE-A- 2 152 142          FR-A- 2 248 015
GB-A- 1 183 497          GB-A- 2 033 233
US-A- 3 272 204          US-A- 4 340 091
US-A- 5 061 276

Remarks:
The file contains technical information submitted
after the application was filed and not included in
this specification

**Description**

BACKGROUND OF THE INVENTION

Field of the Invention:

This invention relates to a novel artificial blood vessel. More particularly, it relates to an artificial blood vessel which features preservation of elasticity and has the ability to resist against crushing and kinking.

Description of the Prior Art:

To date, artificial blood vessels made of stretched polytetrafluoroethylene (produced by Gore Corp., U.S.A. and marketed under trademark designation of "Gore Tex") and those made of polyester (produced by C.R. Bard Corp. and marketed under trademark designation of "Sauvage") have found utility in clinical applications.

These conventional artificial blood vessels are handicapped by poor elasticity and consequent rigidity and, therefore, great difficulties are encountered when they are sutured with vital blood vessels.

It has been reported that since these artificial vessels differ greatly in compliance with vital blood vessels, an artificial blood vessel having a small inside diameter of 6 mm or less, after being buried in a patient's flesh, causes hypertrophy in the sutured section (Trans. Am. Soc. Artif. Interm Organs., 35: 556- (1989)).

The artificial blood vessels made of polyester, for example, which are devoid of elasticity have the disadvantage of producing a kink resembling a collapsed section. For the purpose of preventing this undesirable phenomenon, the practice of crimping the precursory tubes (after the fashion of a bellows) is in vogue. This crimping work, however, has the problem of inevitably sacrificing the smoothness and flatness of the inner surface of the artificial blood vessel (the surface which contacts the blood in motion) which are indispensable for preventing thrombosis.

As a solution for this problem, some of the Sauvage artificial blood vessels made of polyester come in an improved version which avoids the necessity for crimping work and instead requires the peripheral part of the artificial blood vessel to be reinforced with filaments of polypropylene and, because of the reinforcement, has improved resistance to flexure and pressure and enhanced resistance to thrombosis. Since the reinforcing material is lacking in elasticity, the artificial blood vessels of this version suffer not only from drawbacks in terms of physical properties such as the decline in compliance but also from drawbacks in terms of operability such as the necessity of avoiding the parts of the filaments which, during suturing of the artificial blood vessel with a vital blood vessel do not easily allow passage of a needle.

In spite of the desperate need for an artificial blood vessel which is made of a material resembling a vital blood vessel in elasticity, when the artificial blood vessel is vested with this elasticity, it has the disadvantage of being collapsed or bent by external pressure. Conversely, when it incorporates a rigid reinforcing material, it has the same drawbacks in physical properties and operability as observed in the Sauvage artificial blood vessel. None of the artificial blood vessels developed to date have elasticity resembling the due elasticity of a vital blood vessel.

GB-A 1 183 497 discloses a tubular prosthesis adapted to be placed in particular in blood vessels, which comprises an open mesh, non-absorbable, cylindrical tube made of PTFE or polyethylene terephthalate ; and a helical wrapping of polypropylene monofilament around the external wall of the tube.

EP-A-0 448 840 discloses a bicomponent surgical filament useful in a wound closure device which comprises a core having a polymer selected from the group consisting of polyethylene terephthalate and polypropylene, and a sheath comprising a polymer selected from the group consisting of polybutylene terephthalate, polybutester and nylon. It also discloses a surgical filament comprising a blend of polyesters selected from the group consisting of polyethylene terephthalate, polybutylene terephthalate and polybutester.

EP-A-0 552 474, filed on 18.12.92 and published on 28.7.93 relates to an artificial blood vessel comprising a tubular body made of composite fibers composed of a polyethylene terephthalate and a polyester elastic body, said fibers having an elongation rate of not less than 2 % to have an elongation elastic recovery of 90 %. The polyester elastic body may be chosen from the compounds listed on column 5, l. 8-28. The composite fibers may be woven or knitted. If necessary, a spiral or ring reinforcement made of a polyolefin resin may be applied so as to extend along the peripheral surface of the artificial blood vessel.

US 5,061,276 discloses a vascular graft having a composite structure prepared by wrapping about the external surface of a tube having one or more layer of polytetrafluoroethylene or a polytetrafluoroethylene-elastomeric polymer blend, either alone or in combination, an elastic fiber, while maintaining the fiber under tension.

Thus, this invention has as an object thereof the provision of a novel artificial blood vessel.

Another object of this invention is to provide an artificial blood vessel which avoids being collapsed or bent by external pressure and does not cause thrombosis while keeping the same elasticity as a vital blood vessel and, at the same time, facilitates the passing of a needle therethrough during the process of suturing.

## SUMMARY OF THE INVENTION

The objects of the present invention which are described above are accomplished by an artificial blood vessel as specified in Claim 1.

This invention further concerns an artificial blood vessel wherein the artificial blood vessel body is constructed in the shape of a tube fabricated by at least one of the methods of weaving, knitting, and braiding composite fibers made of polyester elastic fibers and polyethylene terephthalate.

We have made a diligent study on artificial blood vessels resembling a vital blood vessel to learn that the objects mentioned above are accomplished by imparting elasticity resembling that of the vital blood vessel both to an artificial blood vessel body and a spiral reinforcing part of the artificial blood vessel. This invention has been perfected based on this knowledge.

The present invention allows production of an artificial blood vessel which excels in resistance to collapsing and kinking, in compliance, and in operability by having an artificial blood vessel body and a reinforcing part thereof both fabricated with materials as described more specifically hereinbelow.

The artificial blood vessel of this invention is endowed with flexibility and elasticity resembling those of a vital blood vessel because the artificial blood vessel body thereof and the reinforcing part of the blood vessel body both have elasticity. Thus, the produced artificial blood vessel acquires physical properties readily adaptable to the human body as evinced by the fact that it permits easy formation of a suture during the process of transplantation and alleviates the damage possibly inflicted on the vital blood vessel by the rigidity of the artificial blood vessel. These characteristic physical properties allow manufacture of an artificial blood vessel body of a small diameter which is able to form a smooth inner surface for flow of blood without necessitating the crimping operation, i.e. a treatment indispensable to the conventional artificial blood vessel of fabric, and consequently exhibits the resistance to thrombosis as required.

## BRIEF DESCRIPTION OF THE DRAWINGS

Figs. 1A - 1E are schematic cross sections each illustrating a composite fiber for forming an artificial blood vessel, Fig. 2 is a schematic diagram illustrating an artificial blood vessel used in a working example as one embodiment of this invention, and

Figs. 3A and 3B are graphs showing the relation between the continuous change of diameter and the flow rate of blood in the artificial blood vessel part used in the working example of this invention.

## DESCRIPTION OF THE PREFERRED EMBODIMENT

Now, the artificial blood vessel body and the reinforcing part of the blood vessel body of this invention will be sequentially described in detail below with reference to working examples.

In the first place, the artificial blood vessel body to be used in this invention is only required to be constructed as specified in Claim 1. Due to the physical properties of the elastic material, the artificial blood vessel body acquires physical properties (flexibility and elasticity) resembling those of the vital blood vessel, allows easy formation of a suture in the process of transplantation, alleviates the damage possibly inflicted on the part of the vital blood vessel used for suturing as a consequence of a mismatch in the compliance between the artificial blood vessel and the vital blood vessel, and exhibits physical properties easily adaptable for the living body. These characteristic physical properties enable the inner surface of the blood vessel for the flow of blood to be smooth and flat without requiring a crimping operation, i.e. a treatment which has been indispensable to the conventional artificial blood vessel formed of a fabric such as of polyester fibers, allow the flow of the blood through the blood vessel to proceed uniformly, and impart improved resistance to thrombosis to the blood vessel.

The fibers to be used in the artificial blood vessel body of this invention are molded in the shape of composite fibers so as to suit the fabrication of an artificial blood vessel body. The term "composite fiber" as used herein refers to a fiber manufactured by causing two or more polymers of differing qualities discharged in independently controlled amounts, combined with one another in one and the same spinneret, and simultaneously spun. It is otherwise called a "conjugate fiber." This fiber is preferred to have undergone conversion into fiber for the purpose of acquiring enhanced elastic recovery. For the sake of stabilizing the tubular construction of the produced artificial blood vessel body, it is preferable to endow it with a latent crimping property after being molded in the shape of fiber. Specifically, the elasticity of the fiber of this invention which has attained an improvement in elastic recovery is preferred to be such that the ratio of elongation exceeds 2% and preferably exceeds 5% and more prefarably exceeds 15 %.

This fiber is preferably given a bulking treatment. The methods which are effectively usable for the bulking treatment include (1) the twisting-heat-setting-untwisting method, (2) the false twisting method, (3) the stuffing method, (4) the abrading method, (5) the air jet method, and (6) the shaping method, for example.

The thickness of this fiber is generally in the range of 10 to 100 deniers, preferably 20 to 50 deniers, and more preferably about 30 deniers. If the thickness of this fiber exceeds 100 deniers, the disadvantage arises that the fiber is notably deprived of elasticity. Conversely, if the thickness of this fiber is less than 10 deniers, the fiber enjoys high elasticity and yet suffers from deficiency in strength.

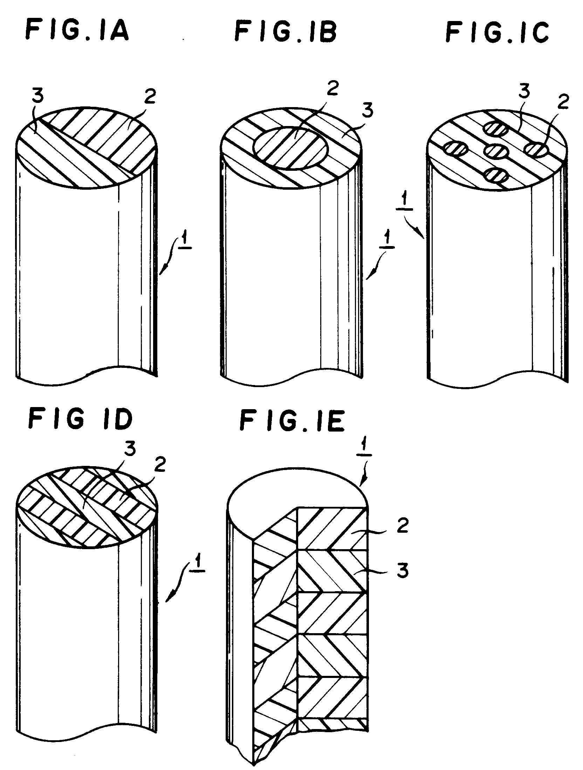

The composite fiber mentioned above can be manufactured in varying forms, depending on the manner in which the component fibers are combined during the process of spinning. Examples of the manner of combination of component fibers are illustrated in Figs. 1A - 1E. Of course, the manners of this combination are not limited to those illustrated herein. The joined type of Fig. 1A: A composite fiber 1 has the two components, i.e. polyethylene terephthalate 2 and a polyester elastomer 3, joined side by side to each other in a mutually applied state. The core and sheath type of Fig. 1B: A composite fiber 1 has a core of one component (polyethylene terephthalate 2) encircled with another component (polyester elastomer 3). The multi-core and mono- sheath type of Fig. 1C: A composite fiber 1 has a multiplicity of core layers of one component (polyethylene terephthalate 2) wholly or virtually wholly enclosed with another component (polyester type elastomer 3). The multilayer joined type of Fig. 1D: A composite fiber 1 has two components, i.e. polyethylene terephthalate 2 and a polyester elastomer 3, alternately joined side by side as applied, fastened mutually in a multiplicity of plies. The axially combined type of Fig. 1E: A composite fiber 1 has polyethylene terephthalate 2 and a polyester elastomer 3 alternately superposed in a multiplicity of layers in the axial direction of fiber.

The joined type of Fig. 1A proves to be particularly preferable for the sake of preserving elasticity and elastic recovery and the manifestation of crimps.

The polyester elastomers which are effectively usable herein include, for example, polybutylene terephthalate, polyester-polyether block copolymer, and polyester-polyester copolymer.

The polyester-polyether block copolymer elastomers include those which have as a hard segment thereof an aromatic polyester such as polyethylene terephthalate, polyethylene terephthalate/isophthalate, or poly(1,4-cyclohexane dimethylene terephthalate) and as a soft segment thereof an aliphatic polyether such as polyethylene glycol. As typical examples of the polyester elastomer, Pelprene-P-type (produced by Toyo Spinning Co., Ltd.) and Arnitel E.P. (produced by Akzo Corp.) may be cited. The polyesterpolyester copolymer elastomers include aliphatic polyesters which have as a hard segment an aromatic polyester such as polyethylene terephthalate, polyethylene terephthalate/-isophthalate, or poly(1,4-cyclohexane dimethylene terephthalate) and as a soft segment an aliphatic polyester such as ethylene sebacate. As a typical example, Pelprene-S-type (produced by Toyo Spinning Co., Ltd.) may be cited.

Though the mixing ratio of the polyethylene terephthalate to the polyester elastomer may be selected at will, it is generally in the range of 20 : 80 to 80 : 20, preferably 40 : 60 to 60 : 40 (by weight). If the proportion of the polyethylene terephthalate is unduly large, the produced composite fiber suffers from a deficiency in the elastic force in spite of an ideal stability of shape. If the balance of the two components is destroyed, a disadvantage arises in that the inclusion of the latent crimping property will possibly fail.

Then, as respects the construction of the artificial blood vessel body of this invention, this blood vessel body is constructed by subjecting the fibers mentioned above to one or more of weaving, knitting, and braiding treatments, for example.

More specifically, the tubular web of the blood vessel body is constructed, for example by warp knitting, weft knitting, triaxial knitting, tubular plain stitching, or knit cording. The construction need not be limited to these methods.

Preferably, in the artificial blood vessel body of this invention which is obtained as described above, countless minute crimps are formed on the inner and outer surface of the tubular web and the tubular web has expansibility resembling that of a vital blood vessel. These minute crimps can be imparted to the tubular web by intertwining the fibers with air jet before the fibers are subjected to the weaving, knitting, or braiding treatment. The expansibility of the artificial blood vessel body having the minute crimps formed thereon is typically such that the elongation in the radial direction is in the range of 2 to 15%, preferably 5 to 15% and the elongation in the axial direction in the range of 2 to 20%, preferably 5 to 15% as measured under an inner pressure load of 199.93 kPa (150 mmHg). When the artificial blood vessel body has the elongation specified above, it enjoys the advantage that the artificial blood vessel transplanted in the living body ideally follows the pulsation of a vital blood vessel and, therefore, manifests outstanding proofness against thrombosis.

The inside diameter of the artificial blood vessel body of this invention can be freely set in a wide range of about 3 to 30 mm, depending on the site of use and the kind of use.

Particularly since the artificial blood vessel body of this invention possesses an extremely preferable ability to remain open, it can fulfill its function sufficiently even when the inside diameter thereof is so small as to fall in a range of about 3 to 6 mm.

Now, the reinforcing part of the artificial blood vessel body constructed of an elastic material contemplated by this invention will be described in detail below. The reinforcing part of the artificial blood vessel is a reinforcing member for the artificial blood vessel fastened to the peripheral surface of the artificial blood vessel body.

The modulus of tensile elasticity of the elastic material is preferred to be in the range of 4.90 to 196.14 MPa (0.5 to 20 kgf/mm$^2$), preferably 9.81 to 98.1 MPa (1 to 10 kgf/mm$^2$).

The elastic material for reinforcement is ethylene-$\alpha$-olefin copolymer or propylene-$\alpha$-olefin copolymer.

The reinforcing part of the artificial blood vessel body applied to the peripheral surface of the artificial blood vessel body is in a helical shape. The reinforcing part used in this shape can impart resistance to kinking of the artificial blood vessel body being used for the application of the reinforcing part, curb the possibility of lowering compliance, and demonstrate an ideal ability to follow changes in outer pressure and changes in inner pressure.

The cross section of the reinforcing part of the artificial blood vessel body perpendicular to the axis of the linear material is not particularly limited to a circle but instead may be a polygon, an ellipse, or a circle containing in the circumference thereof regular rises and falls like a cogwheel. Though the cross section has no particular restriction, it is preferably roundish so as to avoid stimulating the blood vessel.

The outside diameter of such linear materials as filaments forming the reinforcing part of the artificial blood vessel body (the linear materials, although, by way of representation are depicted in this specification as assuming a circular cross section perpendicular to the axis are expected to have a substantially similar thickness in cross section) is freely selected, depending on such factors as the material, physical properties, and outside diameter of the artificial blood vessel body or on the site of use (whether or not the side is an elbow or a knee which by nature is bent frequently). The outside diameter is generally in the range of 0.1 to 2 mm, preferably 0.4 to 1.5 mm, and more preferably 0.5 to 1.0 mm.

As regards the manner of use of the reinforcing part of the artificial blood vessel body, the reinforcing part is applied to the artificial blood vessel body throughout the entire peripheral surface thereof.

The helical reinforcing member is applied to the artificial blood vessel body continuously from the leading end to the trailing end throughout the entire reinforcing region. The helical pitch of this reinforcing part has no particular restriction and is generally in the range of 0.5 to 100 mm, preferably 0.8 to 5 mm.

So long as the pitch is in this range, it is not required to be equal throughout the entire length of the reinforcing region.

The pitch, when necessary, may be decreased in the region corresponding to the site such as an elbow or a knee which by nature is bent frequently.

Now, the formation of the reinforcing part and the method for fast application of the reinforcing part to the peripheral surface of the artificial blood vessel body will be described specifically below.

The formation of the reinforcing part is accomplished, for example, by a method which comprises keeping a metallic bar in rotation round the axis thereof and simultaneously causing the aforementioned elastic material to be linearly discharged as by the injection molding technique and applied helically or annularly to the peripheral surface of the metallic bar.

Subsequently, the reinforcing part obtained as described above is applied fast to the peripheral surface of the artificial blood vessel as by adhesion or welding. When this reinforcing part happens to be made of a polyolefinic thermoplastic elastomer, for example, the fast application of the reinforcing part is attained, for example, by a method which comprises giving to the reinforcing part an inside diameter slightly smaller than the outside diameter of the artificial blood vessel body destined to be furnished with the reinforcing part, forcibly expanding radially the reinforcing part to a diameter slightly larger than the outside diameter of the artificial blood vessel body thereby imparting a thermally shrinking property to the reinforcing part, inserting the artificial blood vessel body into the thermally shrinkable reinforcing part, and heating the reinforcing part thereby causing the reinforcing material to shrink and fasten itself to the artificial blood vessel body; a method which comprises fusing the portion of the reinforcing part destined to contact the artificial blood vessel body or a part of that portion and causing the reinforcing part to be welded to the artificial blood vessel body through the medium of the fused portion; or a method which comprises preparing a solution of the material used for the formation of the reinforcing material in an organic solvent or a solution exhibiting adhesiveness to both the reinforcing material and the material used for the formation of the artificial blood vessel body and applying or spraying the solution onto the reinforcing material deposited on the artificial blood vessel body thereby fastening the reinforcing part to the artificial blood vessel body. Another method which is available for the application under discussion comprises first molding the elastic material in the shape of a rectilinear material, winding the rectilinear material round the artificial blood vessel body, and fastening the material to the vessel body by fusion or by the use of an adhesive liquid.

Still another method which is available for the application comprises causing the material prepared in a molten state for the formation of the reinforcing part to be discharged directly and intermittently onto the peripheral surface of the artificial blood vessel body kept in a rotating state thereby effecting formation and fast deposition of the reinforcing part on the peripheral surface of the artificial blood vessel body.

The method of fast application must be capable of partially, if not wholly, fastening the entire inner wall surface of the reinforcing part to the peripheral surface of the artificial blood vessel body.

The use of the artificial blood vessel of this invention is attained by a method which comprises sterilizing this artificial blood vessel in the patient's living body, and allowing the cells and tissues to adhere thereto or by a method which comprises sterilizing the artificial blood vessel, inoculating thereto in vitro cells collected sterilely from the wall of the host's vital blood vessel, causing the wall surface of the artificial blood vessel to simulate functionally and structurally the vital blood vessel, and transplanting the artificial blood vessel body in the ensuing state to the living body. The artificial blood vessel, when necessary, may be subjected in advance of actual use to any of the various surface treatments heretofore known to the art such as, for example, the treatment with heparin for the impartation of resistance to thrombosis.

Now, this invention will be described more specifically below with reference to working examples. It should be noted, however, that this invention is not limited in any sense to the following examples.

Control 1

Polyethylene terephthalate as one component of a composite fiber and a polyester elastomeric polybutylene terephthalate as the other component thereof were independently melted by means of an extruding device, allowed to flow into a nozzle block set at 290 °C, extruded through a spinneret containing 24 orifices 0.5 mm in diameter by the use of a well-known joined type composite fiber grade nozzle of the front-rear construction at a rate of 20 g/minute per orifice, and rewound at a rate of 1,000 m/minute.

The mixing ratio of the polyethylene terephthalate to the polybutylene terephthalate was 50 : 50 (by weight).

The stretching was carried out at a ratio of 180% between a hot plate set at 80 °C and a hot plate set at 120 °C. Then, the stretched composite fiber was subjected to a bulking treatment by the well-known technique using a twisting speed of 2,500 T/M.

A tubular web was manufactured of the composite fiber by means of a 30-gauge double Russel device. It was produced in a reverse half texture.

Then, the tubular web was turned inside out and subjected to a scouring treatment at 90 °C for 20 minutes in a bath composed of 2 g of soda ash, 2 g of sodium tripolyphosphate, and 2 g of a nonionic surfactant each per liter. This souring treatment deprived the web of impurities and, at the same time, gave rise to crimps.

The artificial blood vessel body consequently produced with an inside diameter of 4 mm, with a stainless steel bar 4 mm in outside diameter inserted through the tube thereof, was heat set at 185°C for 10 minutes, and tested for compliance with vital blood vessels, resistance to collapse under pressure, and resistance to kinking. The results of the test are shown in Table 1. It is clearly noted from Table 1 that this artificial blood vessel body was hardly usable as an artificial blood vessel because it had virtually no resistance to collapsing or kinking.

Example 1:

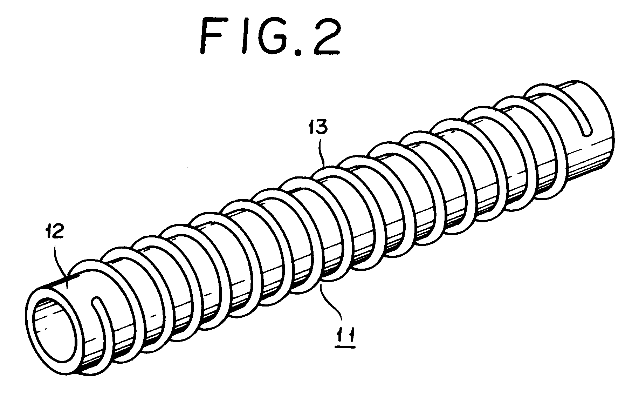

Fig. 2 is a diagram schematically illustrating an artificial blood vessel used in this example.

As illustrated in Fig. 2, an artificial blood vessel 11 of this example was manufactured by causing a filament 0.5 mm in outside diameter of an olefinic elastomer (produced by Mitsui Petrochemical Co., Ltd. and marketed under trademark designation of "TAFMER A20090") as a reinforcing part 13 for an artificial blood vessel body 12 to be helically and continuously wound at a pitch of 1.5 mm round the artificial blood vessel body throughout the entire length thereof, then inserting a stainless steel bar (omitted from illustration) 4 mm in outside diameter through the tube of the artificial blood vessel body, and heating the reinforcing part 13 as deposited on the artificial blood vessel body at 180 °C for 10 minutes thereby fastening by fusion the filament to the artificial blood vessel body 12.

The artificial blood vessel thus obtained was tested for physical properties in the same manner as in Control 1. The results are shown in Table 1. These results indicate that this blood vessel was conspicuously improved in resistance to collapsing and kinking over the blood vessel of Control 1 and was amply suitable for actual use. This product showed no marked loss of compliance.

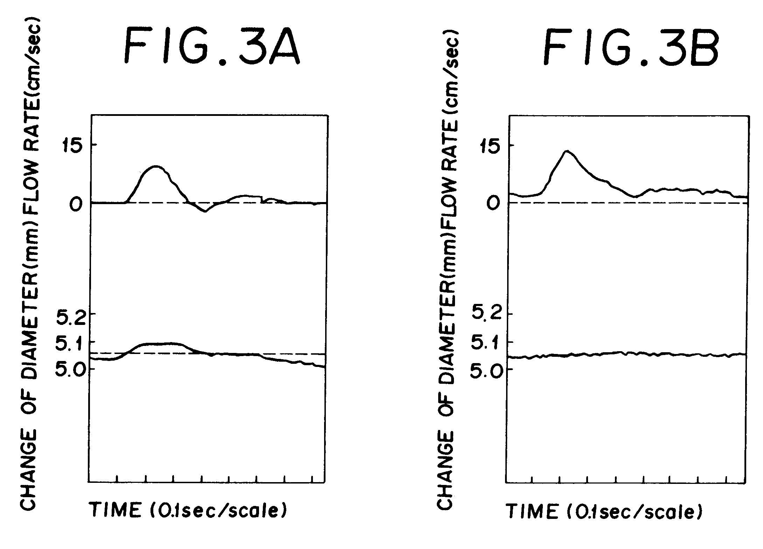

Then, the artificial blood vessel was sterilized with ethylene oxide gas, subsequently subjected in advance of being transplanted to a preclotting treatment with canine blood, and transplanted to the living body of a dog to test for continuous change in diameter of the artificial blood vessel due to the pulsations of a living body and for operability by the technique of suturing of the blood vessels. For the determination of the change in diameter, an ultrasonic blood flow tester (produced by Hayashi Denki K.K. and marketed under product code of "QFM-1100") was used. The results are shown in Fig. 3A. The results clearly indicate that this artificial blood vessel produced a pulsative wave resembling that in the physiological condition. Since the reinforcing part was soft, it offered no obstruction to passage of a needle therethrough and permited easy formation of a suture. Thus, the produced artificial blood vessel permitted a reduction in the time for surgery and an alleviation in surgical infestation.

Control 2

An artificial blood vessel was manufactured by following the procedure of Example 1, except that polypropylene filament was used in the place of the olefinic elastomer filament in the reinforcing part of the artificial blood vessel body.

This artificial blood vessel was tested for physical properties in the same manner as in Control 1. The results are shown in Table 1. These results indicate that this artificial blood vessel showed ideal resistance to collapsing and passable resistance to kinking but had an extreme decline of compliance as compared with the compliance obtained in Example 1.

Table 1

| | Material for reinforcing part of artificial blood vessel body | Strength to resist collapse[1] N/m (gf/cm) | Compliance[2] %/Pa x $10^{-4}$ (%/mmHg) | Radius allowing occurrence of kink (cm) |
|---|---|---|---|---|
| Control 1 | None | 14.71 (15) | 9.75 (0.13) | 5.9 |
| Example 1 | Elastic material (olefinic elastomer filament) | (145) 142.16 | (0.12) 9.00 | 2.3 |
| Control 2 | Nonelastic mateiral (polypropylene filament) | (5050) 4951.02 | (0.09) 6.75 | 1.2 |

Note 1) Strength to resist collapse: This property was determined by compressing a sample artificial blood vessel between two flat surfaces until the distance separating the two flat surfaces reached 80% of the outside diameter of the sample, and measuring the stress prevailing at that time. The magnitude of this property was obtained by dividing the value of the stress by the length of the vessel and reporting the quotient as the strength to resist collapse per unit length.

Note 2) Compliance: This property was determined by measuring the ratio of change of volume at 199.93 kPa (150 mmHg) and dividing the ratio by the volume, as expressed by the formula, $C = \Delta V / \Delta P / V$ (wherein C is compliance, V is volume, and P is pressure).

The artificial blood vessel obtained as described above was subjected to experimental canine transportation in the same manner as in Example 1 to test for the continuous change of diameter of the sample and for operability of the blood vessel suturing technique. The results are shown in Fig. 3B. The results indicate that the sample produced absolutely no pulsative wave even at the maximum blood pressure of 149.28 MPa (112 mmHg) and had a non-physiological behavior. Since the reinforcing part was hard, the sample allowed formation of a suture only with great difficulty.

From the experiments cited above, it is clear that the artificial blood vessel used in Control 2 (widely available at present in the market) acquires outstanding resistance to collapsing when it is reinforced with a polypropylene filament and that the same artificial blood vessel, on being reinforced with such an elastic material as used in Example 1, acquires an ability to recover elastically even when it is collapsed and, therefore, obviates the need for extremely high strength to resist collapsing.

It is further plain from the comparison of pulsative waves that the artificial blood vessel reinforced with a rigid material such as polypropylene as in Control 2 produces no pulsative wave and allows the blood to flow in the nonphysiological condition.

It is also seen that when the same artificial blood vessel is reinforced with an elastic material as in Example 1, it produces a pulsative wave substantially in the physiological condition.

It has been demonstrated that the reinforcing part formed of the elastic material of Example 1 is so soft as to permit easy passage of a needle therethrough and enjoy a notable improvement in operability of the blood vessels suturing technique.

**Claims**

1. An artificial blood vessel (11) comprising an artificial blood vessel body (12) and a helical reinforcing part (13) deposited on said artificial blood vessel body, wherein said body is made of a composite fiber comprising a polyester elastomer and polyethylene terephthalate and said reinforcing part is made of an olefinic elastomer selected from an ethylene-$\alpha$-olefin copolymer and a propylene-$\alpha$-olefin copolymer.

2. An artificial blood vessel according to claim 1, wherein said reinforcing part is made of an ethylene-$\alpha$-olefin copolymer.

3. An artificial blood vessel according to claim 1 or 2, wherein said artificial blood vessel body is constructed in a tubular shape by subjecting fibers to one or more of the weaving, knitting, and braiding treatments.

4. An artificial blood vessel according to any one of claims 1-3, wherein the ratio of elongation required for the ratio of recovery of elasticity from elongation to remain below 90 % is not less than 2 %.

5. An artificial blood vessel according to any one of claims 1-4, wherein the thickness of said fiber is in the range of 10 to 100 deniers.

6. An artificial blood vessel according to any one of claims 1-5, wherein said composite fiber has two components, i.e. polyethylene terephthalate and a polyester elastomer, joined face to face as applied to each other.

7. An artificial blood vessel according to claim 6, wherein said polyester type elastomer is at least one member selected from the group consisting of polybutylene terephthalate, a polyester-polyether block copolymer, and a polyester-polyester copolymer.

8. An artificial blood vessel according to claim 6 or 7, wherein the mixing ratio of said polyethylene terephthalate to said polyester elastomer is in the range of 20 : 80 to 80 : 20.

9. An artificial blood vessel according to any one of claims 1-8, wherein the ratio of elongation in the radial direction is in the range of 2 to 15 % under the load of an inner pressure of 199.93 kPa (150 mmHg).

**Patentansprüche**

1. Künstliches Blutgefäß (11) mit einem künstlichen Blutgefäßkörper (12) und einem auf dem künstlichen Blutgefäßkörper aufgebrachten schraubenförmigen Verstärkungsteil (13), wobei der Körper aus einer ein Polyesterelastomer und Polyethylenterephtalat enthaltenden Verbundfaser hergestellt ist und das Verstärkungsteil aus einem Olefinelastomer hergestellt ist, das aus einem Ethylen-$\alpha$-Olefin-Copolymer und einem Propylen-$\alpha$-Olefin-Copolymer ausgewählt ist.

2. Künstliches Blutgefäß nach Anspruch 1, wobei das Verstärkungsteil aus einem Ethylen-$\alpha$-Olefin-Copolymer hergestellt ist.

3. Künstliches Blutgefäß nach Anspruch 1 oder 2, wobei der künstliche Blutgefäßkorper röhrenförmig aufgebaut ist, indem Fasern einer Web-, Strick- oder Flechtbehandlung oder mehreren dieser Behandlungen unterzogen wurden.

4. Künstliches Blutgefäß nach einem der Ansprüche 1 bis 3, wobei der Dehnungsgrad, der erforderlich ist, damit nach einer Dehnung der Rückstellanteil der Elastizität unter 90% bleibt, nicht weniger als 2% beträgt.

5. Künstliches Blutgefäß nach einem der Ansprüche 1 bis 4, wobei die Dicke der Faser im Bereich von 10 bis 100 Denier liegt.

6. Künstliches Blutgefäß nach einem der Ansprüche 1 bis 5, wobei die Verbundfaser zwei Komponenten, d.h. Polyethylenterephtalat und ein Polyesterelastomer, aufweist, die beim Aufeinanderbringen mit ihren Stirnseiten verbunden wurden.

7. Künstliches Blutgefäß nach Anspruch 6, wobei das Elastomer vom Polyestertyp mindestens ein aus der aus Polybutylenterephtalat, einem Polyester-Polyether-Blockcopolymer und einem Polyester-Polyester-Copolymer bestehenden Gruppe ausgewählter Bestandteil ist.

8. Künstliches Blutgefäß nach Anspruch 6 oder 7, wobei das Mischverhältnis des Polyethylenterephtalats zum Polyesterelastomer im Bereich von 20 : 80 bis 80 : 20 liegt.

9. Künstliches Blutgefäß nach einem der Ansprüche 1 bis 8, wobei der Dehnungsgrad in radialer Richtung unter einer Belastung eines Innendrucks von 199,93 kPa (150 mmHg) im Bereich von 2 bis 15% liegt.

**Revendications**

1. Vaisseau sanguin artificiel (11) comprenant un corps de vaisseau sanguin artificiel (12) et une partie renforçante hélicoïdale (13) déposée sur ledit corps de vaisseau sanguin artificiel, dans lequel ledit corps est constitué par une fibre composite comprenant un élastomère polyester et du polyéthylènetéréphtalate et ladite partie renforçante est constituée par un élastomère oléfinique choisi parmi un copolymère éthylène-$\alpha$-oléfine et un copolymère propylène-$\alpha$-oléfine.

2. Vaisseau sanguin artificiel selon la revendication 1 dans lequel ladite partie renforçante est constituée par un copolymère éthylène-α-oléfine.

3. Vaisseau sanguin artificiel selon la revendication 1 ou 2 dans lequel ledit corps de vaisseau sanguin artificiel est construit sous une forme tubulaire par soumission de fibres à un ou plusieurs des traitements de tissage, tricotage et tressage.

4. Vaisseau sanguin artificiel selon l'une quelconque des revendications 1 à 3 dans lequel le taux d'allongement nécessaire pour que le taux de reprise élastique après allongement reste inférieur à 90 % n'est pas inférieur à 2 %.

5. Vaisseau sanguin artificiel selon l'une quelconque des revendications 1 à 4 dans lequel l'épaisseur de ladite fibre est dans le domaine de 10 à 100 deniers.

6. Vaisseau sanguin artificiel selon l'une quelconque des revendications 1 à 5 dans lequel ladite fibre composite a deux composants, à savoir du polyéthylènetéréphtalate et un élastomère polyester, joints face à face en étant appliqués l'un à l'autre.

7. Vaisseau sanguin artificiel selon la revendication 6 dans lequel ledit élastomère de type polyester est au moins un élément choisi dans le groupe consistant en le polybutylènetéréphtalate, un copolymère séquencé polyester-polyéther et un copolymère polyester-polyester.

8. Vaisseau sanguin artificiel selon la revendication 6 ou 7 dans lequel la rapport de mélange dudit polyéthylènetéréphtalate audit élastomère polyester est dans le domaine de 20 : 80 à 80 : 20.

9. Vaisseau sanguin artificiel selon l'une quelconque des revendications 1 à 8 dans lequel le taux d'allongement dans la direction radiale est dans le domaine de 2 à 15 % sous la charge d'une pression interne de 199,93 kPa (150 mmHg).

FIG.IA

FIG.IB

FIG.IC

FIG ID

FIG.IE

FIG.2

FIG.3A

FIG.3B

EP 0 587 461 B1